# EUROPÄISCHE PATENTANMELDUNG

(11) **EP 1 905 470 A2**
(43) Veröffentlichungstag der Anmeldung: **02.04.2008**
(21) Anmeldenummer: 07018052.6
(22) Anmeldetag: 14.09.2007
(51) Int. Cl.: A61M 15/00, A61M 15/08

(54) **Inhalationsvorrichtung**

(30) Priorität: 28.09.2006 DE 102006047667
(71) Anmelder: Ing. Erich Pfeiffer GmbH, 78315 Radolfzell (DE)
(72) Erfinder: Carrico, Silvio, 78224 Singen (DE); Greiner-Perth, Jürgen, 78244 Gottmadingen (DE); Bommer, René, Dr., 78315 Allensbach-Freudental (DE)
(74) Vertreter: Patentanwälte Ruff, Wilhelm, Beier, Dauster & Partner

(57) **Zusammenfassung**

Die Erfindung betrifft eine Inhalationsvorrichtung (10; 110) zum Austrag eines pulverförmigen Mediums (30; 130) mit mindestens einem Medienspeicher (24; 124), der zur Aufnahme des Mediums (30; 130) vorgesehen ist, und einem Auslasskanal (42; 142) mit einer zumindest in einem Teilabschnitt (46; 146) im Wesentlichen zylindrischen Innenwandung (46; 146), wobei der Auslasskanal (42; 142) als Transportweg für das Medium vom Medienspeicher in eine Atemwegsöffnung eines Benutzers ausgebildet ist und wobei in dem im Wesentlichen zylindrischen Teilabschnitt (46; 146) des Auslasskanals eine Austrittssteueranordnung (60; 160; 260; 360; 460; 560; 660; 760; 860; 960) vorgesehen ist, die als Prallsicherung und/oder Austrittsrichtungsgeber ausgebildet ist.

Verwendung insbesondere für die Applikation von Medikamenten.

## Beschreibung

Die Erfindung betrifft eine Inhalationsvorrichtung zum Austrag eines pulverförmigen Mediums mit mindestens einem Medienspeicher, der zur Aufnahme des Mediums vorgesehen ist, und einem Auslasskanal mit einem im Wesentlichen zylindrischen Teilabschnitt, wobei der Auslasskanal als Transportweg für das Medium vom Medienspeicher in eine Atemwegsöffnung eines Benutzers ausgebildet ist.

Eine solche Inhalationsvorrichtung ist beispielsweise aus der EP 0957962 B1 bekannt. Die dort beschriebene und dargestellte Vorrichtung weist zwei muldenförmige Medienspeicher auf, die vor der Benutzung jeweils mit einer Schutzfolie abgedeckt sind, so dass das Medium nicht entweichen kann. Zur Aktivierung der Inhalationsvorrichtung wird ein Auslasskanal in axialer Richtung in den Medienspeicher eingeschoben, der dabei die Schutzfolie durchdringt. Im Zuge der Inhalation des Mediums wird dieser Auslasskanal anschließend genutzt, um das Medium aus dem Medienspeicher durch Inhalieren in eine Nasenhöhle eines Benutzers zu fördern.

Als nachteilig an diesem Stand der Technik wird angesehen, dass es zu einem versehentlichen Austreten des pulverförmigen Mediums kommen kann, insbesondere im Augenblick des Öffnens der Schutzfolie, und dass das pulverförmige Medium beim Inhalieren aufgrund des relativ großen Durchmessers des Auslasskanals nicht ausreichend gut in eine wunschgemäße Richtung gelenkt werden kann.

### Aufgabe und Lösung

Die Aufgabe der Erfindung besteht darin, eine gattungsgemäße Inhalationsvorrichtung zu schaffen, bei der der Austritt des pulverförmigen Mediums in Hinblick auf die Austrittsrichtung und die Vermeidung verfrühten Austretens verbessert wird.

Erfindungsgemäß wird diese Aufgabe durch eine Inhalationsvorrichtung der Eingangs genannten Art gelöst, bei der in dem im Wesentlichen zylindrischen Teilabschnitt des Auslasskanals eine Austrittssteueranordnung mit mindestens einem Steuerelement angeordnet ist, die als Prallsicherung und/oder Austrittsrichtungsgeber ausgebildet ist.

Eine solche Inhalationsvorrichtung weist ein vorzugsweise mehrteiliges Gehäuse auf, innerhalb dessen der Medienspeicher angeordnet ist und an dem der Auslasskanal so vorgesehen ist, dass er von außen zugänglich ist und eine Inhalation des im Medienspeicher befindlichen pulverförmigen Mediums erlaubt. Die Inhalation erfolgt dabei über den Mund oder die Nase. Bei einer bevorzugten Ausführungsform ist der Medienspeicher vor einer Aktivierung verschlossen, so dass kein Medium austreten kann. Der Auslasskanal weist vorzugsweise vollständig eine im Wesentlichen zylindrische Innenwandung auf, wobei auch Auslasskanäle mit leicht konisch geformten Innenwandungen im Sinne der Erfindung als zylindrisch angesehen werden. In einem zylindrischen bzw. leicht konischen Teilabschnitt des Auslasskanals, vorzugsweise im Bereich einer dem Medienspeicher abgewandten Seite des Auslasskanals, ist die Austrittssteueranordnung vorgesehen, die aus Steuerelementen besteht, die sich in den Querschnitt des zylindrischen Teilabschnitts erstrecken. Diese Steuerelemente können beispielsweise als sich in Querschnittsrichtung erstreckende flächige Leitflächen ausgebildet sein, oder auch als einseitige oder umlaufende Stufen oder Wulste an der Innenwandung. Wenn die Austrittssteueranordnung insbesondere als Prallsicherung dienen soll, sind die Steuerelemente so angeordnet, dass auch bei geöffnetem Medienspeicher, also unmittelbar vor der bestimmungsgemäßen Verwendung der Inhalationsvorrichtung, ein versehentliches Schütteln der Inhalationsvorrichtung oder auch ein Aufwirbeln des pulverförmigen Mediums durch einen kurzen Luftstrom im Bereich des Medienspeichers nicht zu einem Austritt des Mediums aus der Inhalationsvorrichtung führt, da das in den Auslasskanal gelangte Medium gegen die Austrittssteueranordnung stößt und zumindest zum großen Teil dort liegen bleibt oder in den Medienspeicher zurückfällt. Dies ist insbesondere bei Inhalationsvorrichtungen von Vorteil, bei denen das Medium im Lieferzustand durch eine Schutzfolie nach außen abgeschlossen ist und bei denen diese Schutzfolie durch ein Einschieben des Auslasskanals durchbrochen wird. Diesem Durchbrechen geht ein Eindrücken des Auslasskanals in die Schutzfolie voraus, der zu einem Überdruck im Medienspeicher führt und im Augenblick des Eindringens des Auslasskanals ein Aufwirbeln des pulverförmigen Mediums zur Folge haben kann, was bei Fehlen einer Prallsicherung dessen Austreten aus der Inhalationsvorrichtung bewirken kann. Das Aufwirbeln des Mediums kann bei mehrteiligen Gehäusen zusätzlich dadurch begünstigt werden, dass die Gehäuseteile bei der Aktivierung aufeinandertreffen und schlagartig aneinander zum Anliegen kommen, wobei ein Kraftstoß verursacht wird, der impulsartig auf das Medium wirkt. Dient die Austrittssteueranordnung als Austrittsrichtungsgeber, so ist sie vorzugsweise aus Steuerelementen aufgebaut, die dem Luftstrom mit dem pulverförmigen Medium im Zuge der Inhalation im Bereich einer Austrittsöffnung des Auslasskanals ohne wesentliche Erhöhung des Luftwiderstandes eine bestimmte Richtung geben. Dies geschieht vorzugsweise mittels flächiger Leitwerke, deren Flächennormale zu einer Haupterstreckungsrichtung des Auslasskanals einen Winkel zwischen 0 und 45 Grad einschließt.

In einer Weiterbildung der Erfindung ist die Austrittssteueranordnung einstückig an der Innenwandung des Auslasskanals angeformt ist.

Eine solche Ausgestaltung ist besonders in der Herstellung kostengünstig, da nur geringe Modifikationen an der Herstellungsmethode des Auslasskanals vorzunehmen sind. Insbesondere bei einfachen Steueranordnungen, beispielsweise bei Austrittsrichtungsgebern, die lediglich aus einer einfachen, sich in den Auslasskanal erstreckenden Leitfläche bestehen, bietet sich die unmittelbare Anformung an die Innenwandung des Auslasskanals an.

Bei einer alternativen Weiterbildung der Erfindung ist die Austrittssteueranordnung als separates Austrittssteuerbauteil ausgebildet ist, welches in den zylindrischen Teilabschnitt eingesetzt ist.

Eine solche modulare Bauweise, bei der der Auslasskanal und das Austrittssteuerbauteil nicht einstückig ausgeführt sind, bietet sich insbesondere an, wenn beispielsweise abhängig vom verwendeten Medium verschiedene Austrittssteueranordnungen in identischen Auslasskanälen vorzusehen sind. Dadurch, dass die Auslasskanäle für verschiedene Austrittssteueranordnungen verwendbar sind, können sie in großer Stückzahl und dadurch sehr preisgünstig hergestellt werden. Statt den gesamten zylindrischen Teilabschnitt medien- und/oder anwendungsspezifisch anzupassen, kann sich eine solche Anpassung auf das Austrittssteuerbauteil beschränken. Auch unter fertigungstechnischen Gesichtspunkten ist die Auftrennung der Austrittssteueranordnung und des Auslasskanals von Vorteil, da insbesondere bei komplexen Austrittssteueranordnungen die Herstellung der Austrittssteueranordnung als separates Bauteil einfacher ist. Gegebenenfalls kann bei komplexen Austrittssteueranordnungen die separate Fertigung auch die einzige Möglichkeit der Herstellung darstellen.

Die Fixierung des Austrittssteuerbauteils im Auslasskanal kann beispielsweise durch dafür vorgesehene Rastmittel wie Rastnasen und Rastnuten erreicht werden. Eine besonders einfache Form der Fixierung wird erreicht, wenn der Auslasskanal eine leicht konische Innenwandung aufweist, in die das Austrittssteuerbauteil in verjüngende Richtung des Konus eingeschoben wird, so dass es zu einer kraftschlüssigen Verbindung zwischen Austrittsteuerbauteil und Auslasskanal kommt. Neben kraft- und formschlüssigen Verbindungen sind darüber hinaus auch stoffschlüssige Verbindungen wie Klebeverbindungen denkbar und im Einzelfall bevorzugt.

In einer Weiterbildung der Erfindung ist eine Projektion der Austrittssteueranordnung bezogen auf eine Haupterstreckungsachse des zylindrischen Teilabschnitt des Auslasskanals geschlossen.

Unter der Projektion bezogen auf eine Haupterstreckungsachse ist dabei eine Projektion auf eine gedachte Ebene zu verstehen, deren Flächennormale durch die Haupterstreckungsrichtung gebildet wird. Diese Ausprägung der Austrittssteueranordnung führt dazu, dass Pulverpartikel des Mediums, die ungewollt aus dem Medienspeicher in den Auslasskanal geraten, diesen nicht ohne Änderung ihrer Bewegungsrichtung durchqueren können. Ohne einen dafür erforderlichen Trägerluftstrom, der jedoch bei lediglich versehentlichem Schütteln der Inhalationsvorrichtung oder bei kurzen Luftstößen, beispielsweise durch das Aufplatzen der Schutzfolie des Medienspeichers, nicht beziehungsweise nicht in ausreichender Stärke vorliegt, kommt es somit zu keinem Austritt des Mediums. Eine Inhalationsvorrichtung mit einer Austrittssteueranordnung gemäß dieser Weiterbildung stellt daher insbesondere eine gute Prallsicherung dar. Unter einer geschlossenen Projektion wird im Sinne der Erfindung auch eine Projektion verstanden, bei der schmale, linienförmige Freibereich unverschlossen bleiben, da eine lückenlos vollständige Projektion bzgl. der Herstellung sehr hohe Anforderungen stellt. Bei Inkaufnahme von linienförmigen Freibereichen können erheblich günstigere und bzgl. der Werkzeuge verschleissärmere Herstellungsmethoden Anwendung finden.

In einer Weiterbildung der Erfindung weist das Austrittssteuerbauteil einen hohlzylinderförmigen Befestigungsabschnitt auf, der an der Innenwandung des zylindrischen Teilabschnitts des Auslasskanals anliegt.

Der hohlzylinderförmige Befestigungsabschnitt ist dabei an beiden Stirnseiten zumindest teilweise offen. Er ist vorzugsweise so dimensioniert, dass er an der Innenwandung des Auslasskanals derart anliegt, dass er mit diesem eine kraftschlüssige Verbindung bildet. Alternative Ausgestaltungen sehen vor, dass zwischen ihm und der Innenwandung des Auslasskanals eine Spielpassung vorliegt, wobei die Fixierung durch Rastmittel oder eine stoffschlüssige Verbindung geschaffen wird. Die Verwendung eines Austrittssteuerbauteils mit einem hohlzylinderförmigen Befestigungsabschnitt ist insbesondere bezüglich der Handhabung, insbesondere der Montage, vorteilhaft.

Bei einer Weiterbildung der Erfindung weißt die Austrittssteueranordnung mindestens zwei Steuerelemente auf, die bezogen auf eine Haupterstreckungsachse des zylindrischen Teilabschnitts des Auslasskanals in verschiedenen Höhen vorgesehen sind und die vorzugsweise als Leitflächen ausgebildet sind.

Unter verschiedenen Höhen werden in diesem Zusammenhang verschiedene Distanzen verstanden, um die die verschiedenen Steuerelemente von den Enden des Auslasskanals in Haupterstreckungsrichtung beabstandet sind. Das Vorsehen verschiedener Steuerelemente in verschiedener Höhe ermöglicht es, in der Projektion den Querschnitt vollständig zu schließen und dabei dennoch einen weitgehend ungehinderten Luftstrom zuzulassen, der für die bestimmungsgemäße Inhalationsverwendung erforderlich ist. Bei besonderen Anwendungsgebieten kann es zweckmäßig sein, den Abstand zwischen Steuerelementen gering zu halten, um gezielt einen hohen Luftwiderstand zu schaffen. Ein erhöhter Luftwiderstand ist bei Medien zweckmäßig, die idealerweise in nur kleinen Dosen oder mit geringer Geschwindigkeit eingenommen werden. Für eine gezielte Erhöhung des Luftwiderstand kann es sinnvoll sein, mehr als zwei Steuerelemente vorzusehen und diese insbesondere so anzuordnen, dass der Luftstrom mehrmals einer Richtungsänderung unterworfen ist.

In einer Weiterbildung der Erfindung weist das mindestens eine Steuerelement eine kreissegmentförmige oder kreissektorförmige Leitfläche auf.

Solche kreissegmentförmigen oder kreissektorförmigen Leitflächen sind fertigungstechnisch besonders einfach herzustellen. Eine einzelne derartige Leitfläche eignet sich insbesondere als Austrittsrichtungsgeber, während durch mehrere derartige Leitflächen auf verschiedenen Höhen eine konstruktiv einfache Prallsicherung realisierbar ist.

In einer Weiterbildung der Erfindung ist die mindestens eine Leitfläche derart in dem Auslasskanal ausgerichtet, dass ihre Flächennormale mit einer Haupterstreckungsachse des zylindrischen Teilabschnitts einen Winkel größer 0° und kleiner 90° einschließt.

Eine solche als schiefe Ebene ausgebildete Leitfläche stellt eine besonders zweckmäßige und einfache Form eines Austrittsrichtungsgebers dar. Für eine solche Anwendung sind insbesondere Winkel größer 45° von Vorteil, da sie eine wirkungsvolle Umlenkung der Medienpartikel mit einer vergleichsweise geringen Erhöhung des Strömungswiderstandes verbinden. Eine Ausgestaltung mit mehreren Leitflächen, die schräg im Auslasskanal angeordnet sind, ist geeignet, eine vollständig geschlossene Projektion der Austrittssteueranordnung zu realisieren, ohne dass der Strömungswiderstand im Zuge der bestimmungsgemäßen Verwendung der Inhalationsvorrichtung deutlich steigt. Zweckmäßigerweise sind die Leitflächen dabei derart schräg im Auslasskanal angeordnet, dass sie sich von der Innenwandung des Auslasskanals ausgehend zur Mitte des Auslasskanals hin in Richtung einer Austrittsöffnung des Auslasskanals erstrecken.

In einer Weiterbildung der Erfindung ist mindestens eines der Steuerelemente in einer Höhe innerhalb des zylindrischen Teilabschnitts des Auslasskanals vorgesehen, bis zu der sich der hohlzylinderförmige Befestigungsabschnitt nicht erstreckt.

Dies ermöglicht es, eine Prallsicherung zu schaffen, die in Form eines separaten Austrittssteuerbauteils in den Auslasskanal eingefügt wird, ohne zu einer gravierenden Erhöhung des Strömungswiderstandes zu führen. So ist es möglich, eine vollständig geschlossene Projektion der Austrittssteueranordnung dadurch zu schaffen, dass auf der Höhe des hohlzylinderförmigen Befestigungsabschnitts ein erstes Steuerelement vorgesehen ist, welches einen ersten Teil der Querschnittsfläche des Auslasskanals in der Projektion verschließt, während der verbleibende Teil der Projektion durch ein zweites Steuerelement verschlossen wird, welches unterhalb oder oberhalb des hohlzylinderförmigen Befestigungsabschnitts angeordnet ist und mit diesem vorzugsweise durch einen sich in Haupterstreckungsrichtung des Auslasskanals erstreckenden Steg verbunden ist. Das erste Steuerelement auf Höhe des hohlzylinderförmigen Befestigungsabschnitts kann auch durch diesen selbst gebildet sein.

In einer Weiterbildung der Erfindung ist die Austrittssteueranordnung oder ein Teil davon, insbesondere die als Leitflächen ausgebildeten Steuerelemente, elastisch verformbar ausgebildet.

Bei einer solchen Ausgestaltung sind Steuerelemente vorzugsweise bezüglich ihrer Verformbarkeit so ausgelegt, dass sie ohne eine auf sie einwirkende Kraft eine Prallsicherung darstellen, die den Auslasskanal zumindest in einem erheblichen Teilabschnitt verschließt. Wirkt eine ausreichend große Kraft durch den bei der Inhalation verursachten Luftstrom auf die Steuerelemente, so werden sie vollständig oder teilweise verformt, so dass sie der freie Querschnitt im Auslasskanal vergrößert. Dadurch verringert sich der Strömungswiderstand, und der Inhalationsvorgang wird erleichtert. Besonders zweckmäßig ist dabei eine Ausgestaltung, bei der die Leitflächen selbst nicht oder nur in geringem Maße elastisch verformbar sind, jedoch durch Stege mit der Innenwandung des Auslasskanals oder des hohlzylinderförmigen Befestigungsabschnitts verbunden sind, die ihrerseits elastisch verformbar sind und somit in der Art von Schwenkscharnieren funktionieren.

In einer Weiterbildung der Erfindung weist die Austrittssteueranordnung mindestens zwei Steuerelemente auf, wobei das eine Steuerelement eine an der Innenwandung des Auslasskanals oder des Befestigungsabschnitts angeordnete äußere Verjüngung bildet und das andere Steuerelement im Auslasskanal vorzugsweise zentriert angeordnet ist.

Bei dieser Ausgestaltung wird eine vollständig geschlossene oder nahezu vollständig geschlossene Projektion demnach durch zwei Steuerelemente erreicht, wobei das erste Steuerelement einen ringförmigen Außenbereich und das zweite Steuerelement einen darinliegenden Innenbereich der Projektion abdeckt. Während des Inhalationsvorgangs wird der Luftstrom im Bereich des ersten Steuerelements durch die von diesem gebildete innere Öffnung und im Bereich des zweiten Steuerelements allseitig an diesem vorbeigeführt. Eine solche Ausgestaltung der Austrittssteueranordnung weist den besonderen Vorzug auf, dass der Luftstrom nicht in eine bestimmte Richtung innerhalb des Auslasskanals gedrückt wird, sondern homogen und zentriert eine Austrittsöffnung des Auslasskanals durchströmt. Besonders bevorzugt sind Ausführungsformen, bei denen die Steuerelemente eine Form aufweisen, die zu einer Verringerung des Strömungswiderstandes führt. Im Falle des zentrierten Steuerelements kann dies insbesondere durch eine konische Formgebung erreicht werden, wobei der Konus sich entgegen einer Medienaustragsrichtung verjüngt. Als vorteilhaft werden darüber hinaus Ausführungsformen angesehen, bei denen das an der Innenwandung angeordnete Steuerelement unmittelbar durch den hohlzylinderförmigen Befestigungsabschnitt gebildet wird.

In einer Weiterbildung der Erfindung umfasst das mindestens eine Steuerelement einen helixförmigen Kanal, dessen Haupterstreckungsrichtung mit der Haupterstreckungsrichtung des Auslasskanals übereinstimmt.

Eine Austrittssteueranordnung mit einem derartigen helixförmigen Kanal erlaubt in einfacher Art und Weise eine Realisierung einer vollständig geschlossenen Projektion sowie eines Austrittsrichtungsgebers. Hinzu kommt, dass der helixförmige Kanal bei geringem Raumbedarf eine einfache Möglichkeit zur Erzielung einer Homogenisierung des zu inhalierenden Medien-Luft-Gemisches darstellt.

### Kurzbeschreibung der Zeichnungen

Weitere Vorteile und Merkmale der Erfindung ergeben sich aus den Ansprüchen sowie der nachfolgenden Beschreibung mehrerer bevorzugter Ausführungsbeispiele der Erfindung, die anhand der Zeichnungen dargestellt sind. Dabei zeigt:
- Figur 1: eine erfindungsgemäße Inhalationsvorrichtung mit einer besonders einfachen Form einer Austrittssteueranordnung, die unmittelbar an der Wandung des Auslasskanal der Inhalationsvorrichtung angeformt ist,
- Figur 2a bis 2d: die Inhalationsvorrichtung der Fig. 1 in mehreren Stadien der Aktivierung sowie der Verwendung,
- Figur 3: eine zweite Ausführungsform einer erfindungsgemäßen Inhalationsvorrichtung, deren Austrittssteueranordnung mit der der Ausführungsform der Figur 1 übereinstimmt, aber als separates Bauteil realisiert ist,
- Figur 4 bis 11: verschiedene Varianten der Austrittssteueranordnung in jeweils unterschiedlichen Darstellungen und
- Figur 12: eine dritte Ausführungsform einer erfindungsgemäßen Inhalationsvorrichtung, bei der eine Austrittssteueranordnung und eine Spitze zum Öffnen eines Medienspeichers als gemeinsames, einstückiges Bauteil ausgeführt sind.

### Detaillierte Beschreibung der Ausführungsbeispiele

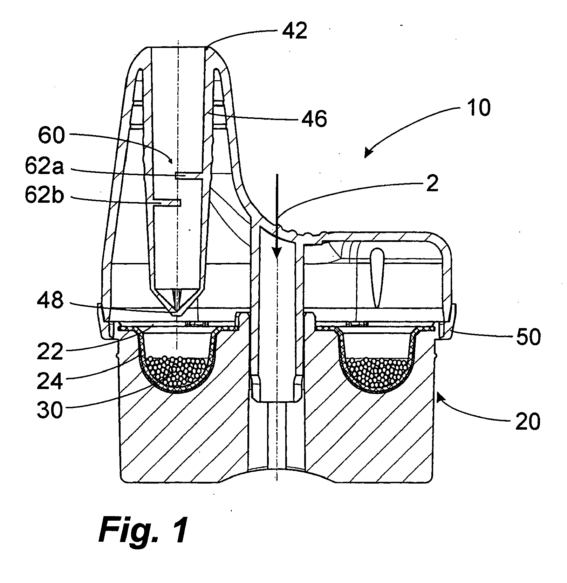

Figur 1 zeigt eine erste Ausführungsform einer erfindungsgemäßen Inhalationsvorrichtung 10, die zusammengesetzt ist aus einem unteren Gehäuseteil 20 sowie einem oberen Gehäuseteil 40. Im unteren Gehäuseteil 20 sind zwei Medienspeicher 24 vorgesehen, die jeweils eine bestimmte Menge eines pulverförmigen Mediums 30 enthalten. Die muldenförmigen Medienspeicher 24 sind durch Schutzfolien 22 abgedeckt und gegenüber der Umgebung verschlossen. Das obere Gehäuseteil 40 weist einen Auslasskanal 42 auf, dessen Innenwandung 46 sich in Richtung des Medienspeichers 24 konisch verjüngt.

Die obere Gehäusehälfte 40 ist in eine Fügerichtung 2 gegenüber dem unteren Gehäuseteil 20 verschiebbar ausgebildet, was dem Zweck dient, mit einer an einem inneren Wandungsabschnitt 46 angeformten Spitze 48 des Auslasskanals 42 die Schutzfolie 22 aufzustechen. Bei dem in Figur 1 dargestellten Zustand wird eine Relativverschiebung allerdings durch einen entfernbaren Sicherheitsring 50 blockiert, der als Originalitätsschutz dient und ein versehentliches Aktivieren der Inhalationsvorrichtung 10 verhindert.

Im Auslasskanal 42 ist innerhalb des inneren Wandungsabschnitts 46 eine Austrittssteueranordnung 60 angeordnet, die aus zwei jeweils in etwa halbkreisförmigen Leitflächen 62a, 62b besteht, die in verschiedener Höhe auf gegenüberliegenden Seiten des inneren Wandungsabschnitts 46 des Auslasskanals 42 angeformt sind.

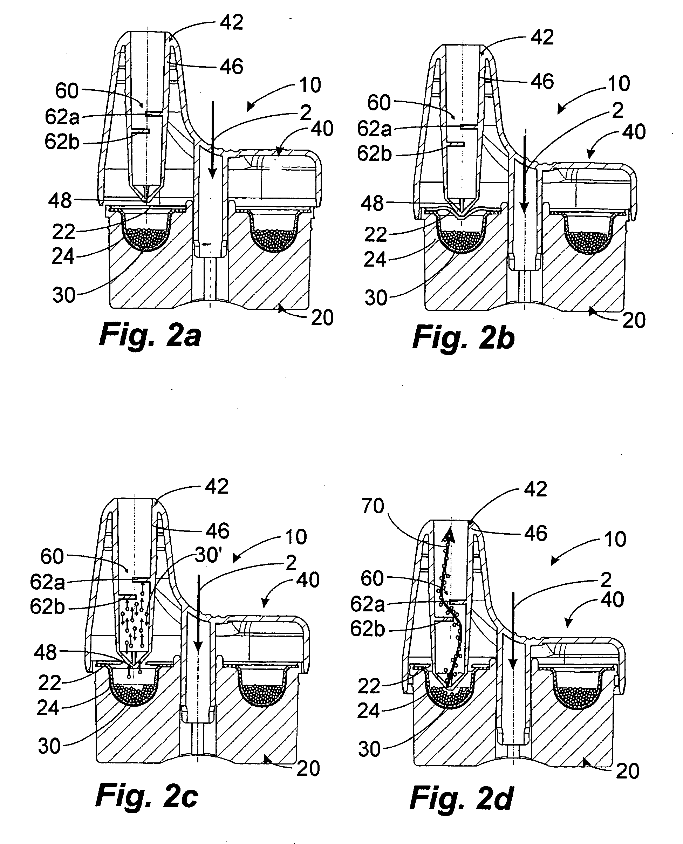

Die Figuren 2a bis 2d stellen die Funktionsweise der Inhalationsvorrichtung 10 und insbesondere der Austrittssteueranordnung 60 dar. Ausgehend von dem Ursprungszustand, der in Figur 1 dargestellt ist, können nach Entfernen des Sicherheitsrings 50 - dargestellt in Figur 2a - die beiden Gehäuseteile 20, 40 ineinander gedrückt und die Inhalationsvorrichtung damit aktiviert werden.

Wie der Figur 2b zu entnehmen ist, wird dabei die untere Spitze 48 des inneren Wandungsabschnitts 46 auf die Schutzfolie 22 des einen Medienspeichers 24 gedrückt. Die Schutzfolie 24 verformt sich dabei und im Medienspeicher 22 kommt es zu einem Überdruck.

Das Eindrücken wird solange fortgesetzt, bis - wie in Figur 2c dargestellt ist - die Schutzfolie 24 zerreist. Die dadurch aus dem Medienspeicher 22 entweichende Luft reißt einen geringen Teil 30' des pulverförmigen Mediums 30 mit sich. Dieser Teil 30' gelangt in den Auslasskanal 42. Der Effekt wird durch die Verdrängung der Luft aus der oberen Gehäusehälfte 40 noch verstärkt, da diese zum Teil ebenfalls durch den Medienspeicher 22 hindurch in den Auslasskanal 42 entweicht. Der in den Auslasskanal 42 gelangte Teil 30' des pulverförmigen Mediums 30 kann aufgrund der Austrittssteueranordnung 60 jedoch nicht durch den Auslasskanal 42 nach außen austreten. Stattdessen prallen die Pulverpartikel 30' zu einem überwiegenden Teil gegen die Leitflächen 62a, 62b und fallen in den Medienspeicher 24 zurück.

Erst wenn nach Ansetzen des Auslasskanals 42 an ein Nasenloch des Benutzers dieser durch Einatmen einen kontinuierlichen lnhalationsluftstrom 70 erzeugt, kann das pulverförmige Medium 30, das durch den Luftstrom 70 mitgerissen wird, an den Leitflächen 62a, 62b vorbei aus dem Austrittskanal 42 nach oben austreten.

Die dargestellte Austrittssteueranordnung führt also dazu, dass ein Austrag nur dann möglich ist, wenn ein durch Einatmen verursachter kontinuierlicher Luftstrom als Träger des pulverförmigen Mediums dieses herausbefördert. Ein nur impulsartiger kurzer Luftstoß, wie er beispielsweise durch das Zerreißen der unter Überdruck stehenden Schutzfolie 24 hervorgerufen wird, reicht nicht, um das pulverförmige Medium an der Austrittssteueranordnung vorbei nach außen zu fördern. Ebenso ist im Zeitraum nach Aktivierung und vor Benutzung ein Austrittsschutz gewährleistet, wenn die Inhalationsvorrichtung 10 versehentlich ruckartig bewegt wird. Der durch die Massenträgheit gegebenenfalls in den Austrittskanal 42 geratene Teil des Mediums 30 wird von den Leitflächen 62a, 62b weit überwiegend an einem Austreten gehindert.

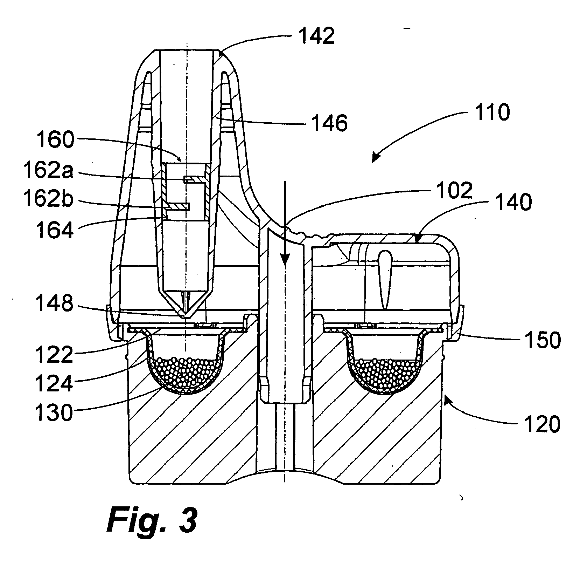

Figur 3 zeigt eine alternative Ausführungsform, die sich lediglich dadurch von der Ausführungsform der Figur 1 unterscheidet, dass die Austrittssteueranordnung als separates Austrittssteuerbauteil 160 vorliegt. Dieses Austrittssteuerbauteil 160 besteht aus einem Hohlzylinder 164, der bezüglich seines Außendurchmessers dem Innendurchmesser der Innenwandung 146 des Auslasskanals 142 in etwa entspricht, sowie aus Leitflächen 162a, 162b, die bis auf eine geringfügig kleinere Fläche den Leitflächen 62a, 62b der Ausführungsform der Figur 1 entsprechen. Die Funktionsweise dieser zweiten Ausführungsform ist mit der Funktionsweise der ersten Ausführungsform der Figur 1 identisch. Unterschiede ergeben sich lediglich in Hinblick auf die Herstellung sowie auf die durch den geringeren Querschnitt des Hohlzylinders 164 gegenüber dem Auslasskanal 42 verursachten konkreten Strömungsbedingungen. Bei der Herstellung werden das obere Gehäuseteil 140 und das Austrittssteuerbauteil 160 separat hergestellt und anschließend zusammengefügt. Diese modulare Bauweise ist insbesondere dann vorzuziehen, wenn eine Vielzahl verschiedener Austrittssteueranordnungen hergestellt werden soll, die allesamt in ansonsten baugleichen oberen Gehäuseteilen 140 Anwendungen finden sollen, oder wenn das obere Gehäuseteil 140 sowohl für Inhalationsvorrichtungen mit und ohne Austrittssteuerbauteil 160 verwendbar sein soll.

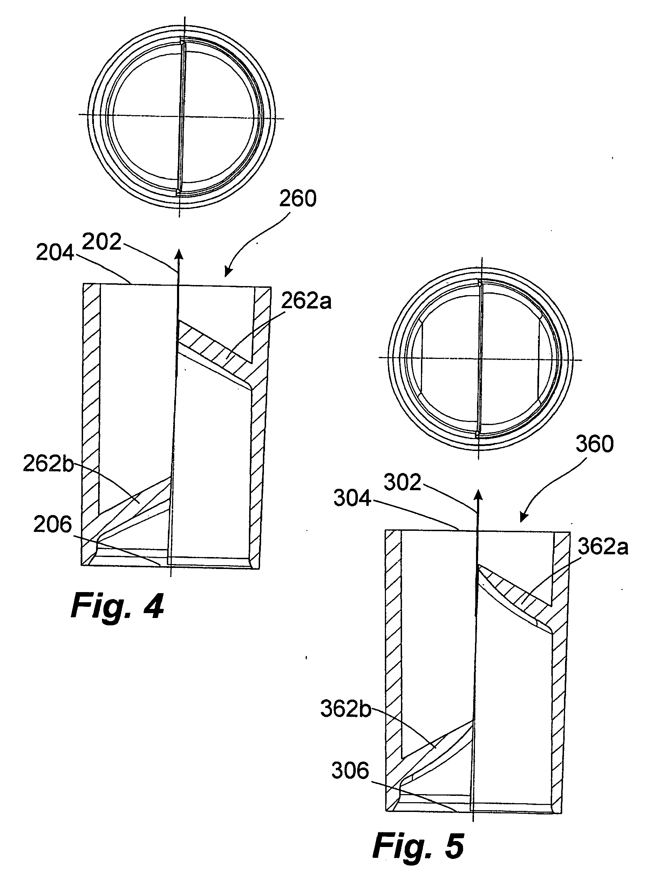

Die Figuren 4 bis 11 zeigen verschiedene vorteilhafte Austrittssteueranordnungen, wobei diese Geometrien jeweils als separate Austrittssteuerbauteile realisiert sind. Es wird jedoch darauf hingewiesen, dass die Austrittssteueranordnungen gleichermaßen auch als mit dem Auslasskanal einstückig und an dessen Innenwandung angeformt sein können.

Die Figuren 4 bis 6 zeigen jeweils Austrittssteuerbauteile 260, 360, 460 mit jeweils zwei als Leitflächen ausgebildeten Steuerelementen 262a, 262b, 362a, 362b, 462a, 462b. Die Ausführungsformen der Figuren 4 und 5 unterscheiden sich von der Ausführungsform der Figur 1 dadurch, dass die Leitflächen 262a, 262b, 362a, 362b jeweils so im Auslasskanal angeordnet sind, dass sie zur Auslasskanalmitte hin in Richtung einer Auslassseite 204, 304 ansteigen. Hierdurch wird eine Verminderung des Strömungswiderstandes gegenüber der Ausführungsform der Figur 1 erreicht. Dies erleichtert den Inhalationsvorgang, ohne die Eignung der Austrittssteuerbauteile 260, 360 in Hinblick auf ihre Funktion als Prallsicherung nennenswert zu verringern. Die beiden Ausführungsformen der Figuren 4 und 5 unterscheiden sich voneinander dadurch, dass sich die Leitflächen 362a, 362b des Austrittssteuerbauteils 360 zur Mitte verjüngen und somit einen geringeren Strömungswiderstand hervorrufen.

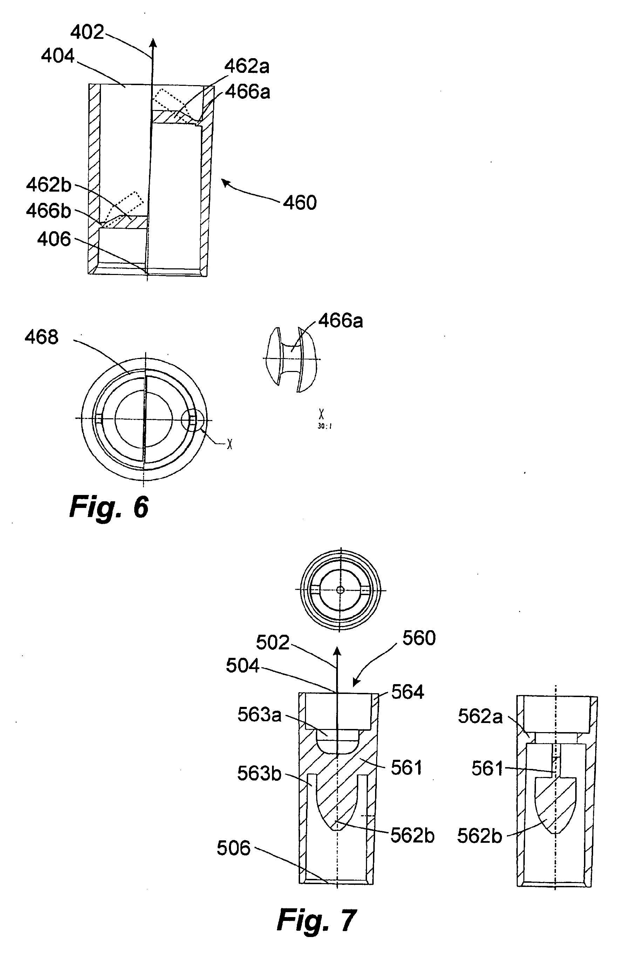

Die Ausführungsform der Figur 6 ähnelt den vorgenannten Ausführungsformen insofern, als dass ebenfalls zwei etwa halbkreisförmige Leitflächen 462a, 462b als Steuerelemente vorgesehen sind. Abweichend von den vorgenannten Ausführungsformen sind diese Leitflächen 462a, 462b jedoch nur mittels eines schmalen und gegenüber der Dicke der Leitflächen verjüngten Stegs 466a, 466b mit dem Hohlzylinder 464 verbunden. In einem umlaufenden Bereich 468, in dem die Leitflächen 462a, 462b nicht mit dem Hohlzylinder 464 verbunden sind, ergibt sich somit ein freier schmaler Spalt 468. Aufgrund der geringen Breite des Spaltes 468 ist die Wirkung der Austrittssteueranordnung 460 als Prallsicherung nicht wesentlich verringert. Die Anbindung der Leitflächen 462a, 462b an den Hohlzylinder mittels schmaler Stege 466a, 466b führt jedoch in vorteilhafter Art und Weise dazu, dass im Zuge des Inhalationsvorgangs die Leitflächen durch die Sogwirkung 462a, 462b in Austrittsrichtung 404 ausgelenkt werden. Die ausgelenkte Stellung ist in Figur 6 gestrichelt dargestellt. Die Verringerung des Luftwiderstandes, die durch die Auslenkung erzielt wird, erleichtert dem Benutzer den Inhalationsvorgang.

Die Figuren 7 bis 10 zeigen Austrittssteueranordnungen, die abweichend von den vorangegangenen Ausführungsbeispielen keine kreissegmentförmigen Leitflächen aufweisen, sondern stattdessen ein erstes, am Rand angeordnetes, umlaufendes Steuerelement und ein zweites, zentrisch angeordnetes Steuerelement, die gemeinsam ebenfalls eine Prallsicherung bilden.

Bei der Ausführungsform der Figur 7 ist innerhalb eines Hohlzylinders 564 das erste Steuerelement 562a als ein an der Innenseite des Hohlzylinders 564 angeformter Ring mit etwa quadratischem Querschnitt ausgebildet. Dieses erste Steuerelement 562a umschließt eine Öffnung 563a. Das zweite Steuerelement 562b ist unterhalb des ersten Steuerelements 562a zentrisch innerhalb des Hohlzylinders 564 angeordnet und hat eine sich konisch in Richtung der Eintrittseite 506 verjüngende Form. Zwischen dem zweiten Steuerelement 562b, dessen Durchmesser dem Durchmesser der Öffnung 563a entspricht, und dem Hohlzylinder 564 liegt ein Durchgangsspalt 563b. Das zweite Steuerelement 562b ist über schmale Stege 561 mit dem Hohlzylinder 564 verbunden.

Auch diese Austrittssteueranordnung der Figur 7 weist eine nahezu vollständig geschlossene Projektion auf, so dass ohne einen kontinuierlichen Luftstrom auf der Eintrittsseite 506 eintretendes pulverförmiges Medium vom ersten Steuerelement 563a oder dem zweiten Steuerelement 563b am Austritt an der Austrittsseite 504 gehindert wird. Beim Vorliegen eines durch die Inhalation verursachten Luftstroms kann das pulverförmige Medium jedoch durch den Durchgangsspalt 563b und die Durchgangsöffnung 563a hindurch von der Eintrittsseite 506 zur Austrittsseite 504 gelangen.

Vorteilhaft an dieser Ausgestaltung gegenüber den Ausführungsformen der Figuren 1 bis 6 ist, dass durch die weitgehend rotationssymetrische Anordnung der Steuerelemente die Austrittsrichtung des pulverförmigen Mediums in etwa der Haupterstreckungsrichtungen des Auslasskanals entspricht. Dies ist insbesondere bei solchen Inhalationsvorrichtung von Vorteil, die nicht zwingend in einer definierten Stellung verwendet werden.

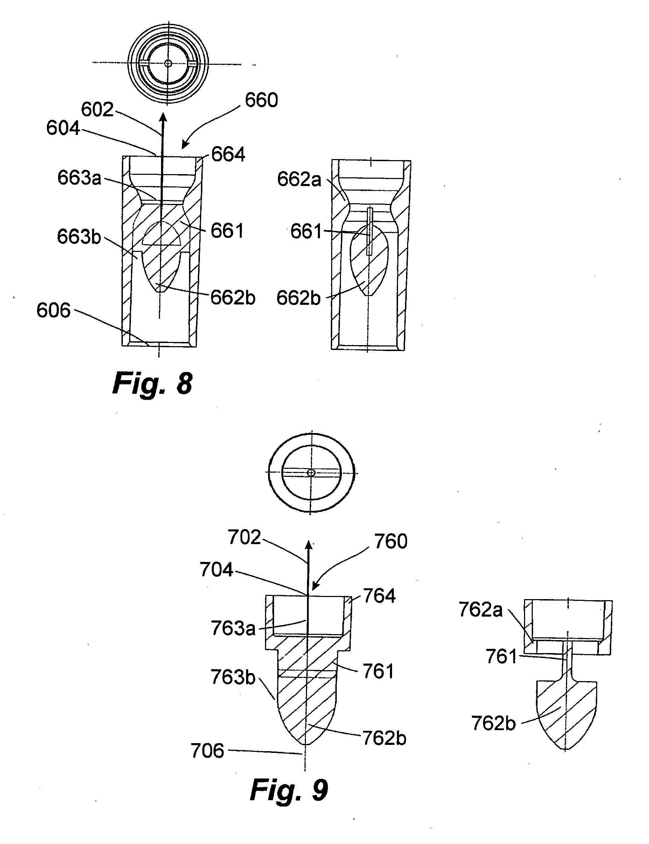

Die Ausführungsform der Figur 8 entspricht weitgehend der Ausführungsform der Figur 7. Abweichend von dieser ist das erste Steuerelement 662a jedoch als einfache Verjüngung des Hohlzylinders 664 ausgebildet. Scharfe Kanten, die strömungstechnisch in Hinblick auf die für den Inhalationsvorgang erforderliche Sogwirkung störend sind, sind dabei nicht vorhanden.

Die Figur 9 zeigt eine weitere Ausführungsform eines Austrittssteuerbauteils 760. Dieses ähnelt dem Austrittssteuerbauteil 560 der Figur 7. Abweichend von diesem weist es jedoch einen deutlich verkürzten Hohlzylinder 764 auf, der sich nicht bis in den Bereich des zweiten Steuerelements 762b erstreckt. Darüber hinaus ist das erste Steuerelement 762a lediglich als nur geringfügige Verbreiterung der Wandstärke des Hohlzylinders 764 ausgebildet. Vorteilhaft an dieser Ausführungsform ist, dass der Durchmesser des Auslasskanals in den das Austrittssteuerbauteil 760 eingesetzt wird, nicht durch den Hohlzylinder 764 auf ganzer Länge verringert wird, wie es bei den Ausführungsformen der Fig. 7 und 8 der Fall ist. Verbesserte Strömungseigenschaften mit einem geringeren Strömungswiderstand während des Inhalationsvorgangs sind die Folge.

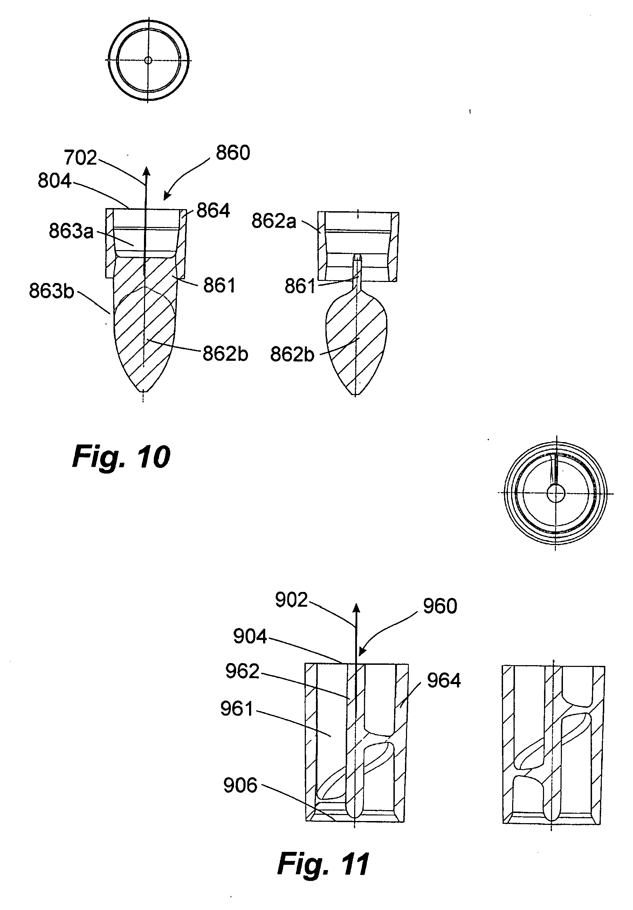

Die Figur 10 zeigt eine noch weitere Ausführungsform, die diesen vorteilhaften Effekt ebenfalls ausnutzt. Gegenüber der Ausführungsform der Figur 9 bildet hierbei jedoch der Hohlzylinder 864 unmittelbar selbst das erste Steuerelement 862a.

Figur 11 zeigt eine davon deutlich abweichende weitere Ausführungsform einer Austrittssteueranordnung. Bei dieser ist innerhalb des Hohlzylinders 960 als Steuerelement 962 eine Struktur mit einem helixförmigen Durchgangskanal 961 ausgebildet. Dieses Steuerelement 962 stellt ebenfalls eine geschlossene Projektion dar und ist damit ebenfalls als Prallsicherung geeignet. Darüber hinaus bietet eine derartige Ausführungsform den Vorteil, dass es innerhalb des Durchgangskanals 961 zu einer Verwirbelung und damit zu einer vorteilhaften Homogenisierung des zu inhalierenden Luft-Medien-Gemisches kommt.

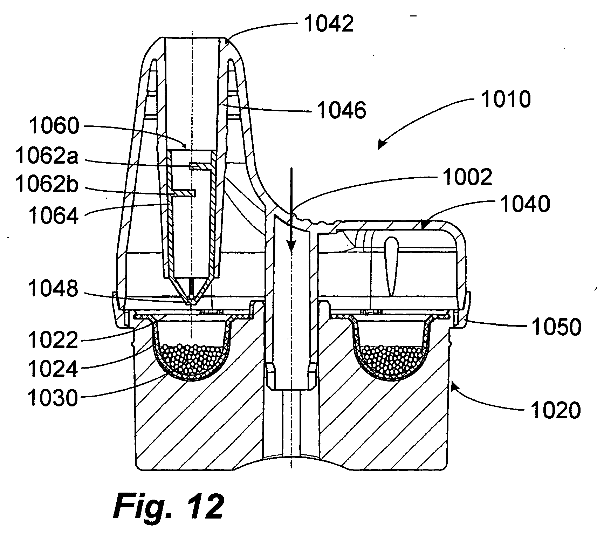

Die Ausführungsform der Figur 12 ähnelt der Ausführungsform der Figur 3. Abweichend ist die Spitze 1048 jedoch nicht am zylindrischen Abschnitt 1046 des Auslasskanals 1042 angeformt, sondern bildet zusammen mit der Austrittssteueranordnung 1060 ein einstückiges Bauteil, welches kraftschlüssig im Auslasskanal 1042 befestigt ist. Eine derartige Gestaltung erhöht die Flexibilität in Hinblick auf die Gestaltung des Auslasskanals und der Austrittssteueranordnung.

## Patentansprüche

1. Inhalationsvorrichtung (10) zum Austrag eines pulverförmiges Mediums (30) mit
- mindestens einem Medienspeicher (24), der zur Aufnahme des Mediums (30) vorgesehen ist, und
- einem Auslasskanal (42) mit einem im Wesentlichen zylindrischen Teilabschnitt (46),
wobei der Auslasskanal (42) als Transportweg für das Medium vom Medienspeicher (30) in eine Atemwegsöffnung eines Benutzers ausgebildet ist,
**dadurch gekennzeichnet, dass**
in dem im Wesentlichen zylindrischen Teilabschnitt (46) des Auslasskanals (42) eine Austrittssteueranordnung (60; 160; 260; 360; 460; 560; 660; 760; 860; 960) mit mindestens einem Steuerelement (62a, 62b; 162a, 162b; 262a, 262b; 362a, 362b; 462a, 462b; 562a, 562b; 662a, 662b; 762a, 762b; 862a, 862b; 962) angeordnet ist, die als Prallsicherung und/oder Austrittsrichtungsgeber ausgebildet ist.

2. Inhalationsvorrichtung nach Anspruch 1,
**dadurch gekennzeichnet, dass**
die Austrittssteueranordnung (60) einstückig an einer Innenwandung des Auslasskanals (42) angeformt ist.

3. Inhalationsvorrichtung nach Anspruch 1,
**dadurch gekennzeichnet, dass**
die Austrittssteueranordnung (160; 260; 360; 460; 560; 660; 760; 860; 960) als separates Austrittssteuerbauteil (160; 260; 360; 460; 560; 660; 760; 860; 960) ausgebildet ist, welches in den zylindrischen Teilabschnitt (146) eingesetzt ist.

4. Inhalationsvorrichtung nach einem der vorstehenden Ansprüche,
**dadurch gekennzeichnet, dass**
eine Projektion der Austrittssteueranordnung (60; 160; 260; 360; 460; 560; 660; 760; 860; 960) bezogen auf eine Haupterstreckungsachse (2; 102; 202; 302; 402; 502; 602; 702; 802; 902) des zylindrischen Teilabschnitt (46; 146) des Auslasskanals (42; 142) geschlossen ist.

5. Inhalationsvorrichtung nach Anspruch 3 oder 4,
**dadurch gekennzeichnet, dass**
das Austrittssteuerbauteil (160; 260; 360; 460; 560; 660; 760; 860; 960) einen hohlzylinderförmigen Befestigungsabschnitt (164; 264; 364; 464; 564; 664; 764; 864; 964) aufweist, der an der Innenwandung des zylindrischen Teilabschnitts (146) des Auslasskanals (142) anliegt.

6. Inhalationsvorrichtung nach einem der vorstehenden Ansprüche,
**dadurch gekennzeichnet, dass**
die Austrittssteueranordnung (60; 160; 260; 360; 460; 560; 660; 760; 860) mindestens zwei Steuerelemente (62a, 62b; 162a, 162b; 262a, 262b; 362a, 362b; 462a, 462b; 562a, 562b; 662a, 662b; 762a, 762b; 862a, 862b) aufweist, die bezogen auf eine Haupterstreckungsachse (2; 102; 202; 302; 402; 502; 602; 702; 802; 902) des zylindrischen Teilabschnitts (46; 146) des Auslasskanals (42; 142) in verschiedenen Höhen vorgesehen sind und die vorzugsweise als Leitflächen (62a, 62b; 162a, 162b; 262a, 262b; 362a, 362b; 462a, 462b) ausgebildet sind.

7. Inhalationsvorrichtung nach einem der vorstehenden Ansprüche,
**dadurch gekennzeichnet, dass**
das mindestens eine Steuerelement (62a, 62b; 162a, 162b; 262a, 262b; 362a, 362b; 462a, 462b) eine kreissegmentförmige oder kreissektorförmige Leitfläche aufweist (62a, 62b; 162a, 162b; 262a, 262b; 362a, 362b; 462a, 462b).

8. Inhalationsvorrichtung nach Anspruch 7,
**dadurch gekennzeichnet, dass**
die mindestens eine Leitfläche (262a, 262b; 362a, 362b; 462a, 462b) derart in dem Auslasskanal ausgerichtet ist, dass ihre Flächennormale mit einer Haupterstreckungsachse (202, 302, 402) des zylindrischen Teilabschnitts einen Winkel größer 0° und kleiner 90° einschließt.

9. Inhalationsvorrichtung nach einem der Ansprüche 5 bis 8,
**dadurch gekennzeichnet, dass**
mindestens eines der Steuerelemente (762b; 862b) in einer Höhe innerhalb des zylindrischen Teilabschnitts des Auslasskanals vorgesehen ist, bis zu der sich der hohlzylinderförmige Befestigungsabschnitt (764; 864) nicht erstreckt.

10. Inhalationsvorrichtung nach einem der vorstehenden Ansprüche,
**dadurch gekennzeichnet, dass**
die Austrittssteueranordnung (460) oder ein Teil (462a, 462b) davon, insbesondere die als Leitflächen (462a, 462b) ausgebildeten Steuerelemente (462a, 462b), elastisch verformbar ausgebildet ist.

11. Inhalationsvorrichtung nach einem der vorstehenden Ansprüche,
**dadurch gekennzeichnet, dass**
die Austrittssteueranordnung (560; 660; 760; 860) mindestens zwei Steuerelemente (562a, 562n; 662a, 662b; 762a, 762b; 862a, 862b) aufweist, wobei das eine Steuerelement (562a; 662a; 762a; 862a) eine an der Innenwandung des Auslasskanals oder des Befestigungsabschnitts (564; 664; 764; 864) angeordnete äußere Verjüngung bildet und das andere Steuerelement (562b; 662b; 762b; 862b) im Auslasskanal vorzugsweise zentriert angeordnet ist.

12. Inhalationsvorrichtung nach einem der vorstehenden Ansprüche,
**dadurch gekennzeichnet, dass**
das Steuerelement (962) einen helixförmigen Kanal umfasst, wobei die Haupterstreckungsrichtung (902) der Helix mit der Haupterstreckungsrichtung des Auslasskanals übereinstimmt.
